(19) Europäisches Patentamt
European Patent Office
Office européen des brevets

(11) Publication number: **0 152 825**
**B1**

(12) # EUROPEAN PATENT SPECIFICATION

(45) Date of publication of patent specification: **17.05.89**

(21) Application number: **85100931.6**

(22) Date of filing: **30.01.85**

(51) Int. Cl.[4]: **C 08 L 69/00,** C 08 L 67/02, A 61 L 31/00, A 61 L 29/00 // A61L2/08 ,(C08L69/00, 67:02, 69:00),(C08L67/02, 67:02, 69:00)

(54) **Use of a polymer blend for producing medical devices with improved resistance to yellowing under ionizing radiation.**

(30) Priority: **10.02.84 US 579102**

(43) Date of publication of application:
**28.08.85 Bulletin 85/35**

(45) Publication of the grant of the patent:
**17.05.89 Bulletin 89/20**

(84) Designated Contracting States:
**DE FR GB IT NL**

(56) References cited:
**WO-A-83/01255**
**DE-A-2 750 062**
**FR-A-2 128 704**
**GB-A- 951 818**
**US-A-4 414 230**

**N.SZYCHER: "Biocompatible Polymers, Metals and Composites", 18th March 1983, pages 975-1018, Chapter 43: H.LANDFIELD: "Sterilization of medical devices based on polymer selection and stabilization techniques" and Chapter 44: W.ESKIENS et al.:**

(73) Proprietor: **GENERAL ELECTRIC COMPANY**
**1 River Road**
**Schenectady New York 12305 (US)**

(72) Inventor: **Allen, Richard Brian**
**525 Windsor Road**
**Dalton Massachusetts 01226 (US)**
Inventor: **Avakian, Roger W.**
**212 Doreen Street**
**Pittsfield Massachusetts 01201 (US)**

(74) Representative: **Schüler, Horst, Dr. et al**
**Kaiserstrasse 69**
**D-6000 Frankfurt/Main 1 (DE)**

(56) References cited:
**"Ionizing radiation's effects on selected biomedical polymers", Technomic Publishing Co., Lancaster, GB;**

**CHEMICAL ABSTRACTS, vol. 82, no. 16, 21st April 1975, page 70, no. 99299g, Columbus, Ohio, US; & JP - 74 115 146 (TEIJIN CHEMICALS LTD.) 02-11-1974**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 152 825 B1

## Description

Polymers which can be fabricated into transparent plastic articles, for example, aromatic polycarbonate resins and blends, are sometimes used in products for the medical field. These products include blood oxygenators, anesthesia canisters, intravenous connectors and accessories, pulsatile balloon pumps, and blood centrifuge banks. Such articles are frequently sterilized, typically by heating in an autoclave, or by contacting with ethylene oxide, or by exposure to ionizing radiation, e.g., gamma radiation or electron beam radiation. Each of these techniques has certain shortcomings, however. Autoclaves are often undesirable because of the thermal instability of many polymers, including polycarbonates and polyarylates, the relatively high energy requirements of the technique, and the residual wetness of the treated article which must first be dried before use.

The utilization of ethylene oxide is often objectionable because of its toxicity, instability and the environmental concerns associated with its disposal.

Sterilization by ionizing radiation is a useful alternative, being an essentially dry process which can be conducted at low temperatures and which is relatively inexpensive. The use of ionizing radiation when applied to articles made of polycarbonate and polyarylate resins in particular usually results, however, in the formation of a yellow coloration in the normally optically clear polymer. This can be regarded as unsightly, and to counteract the effect coloring agents have often been incorporated into the polymer to mask the yellow color which forms with one considered more esthetically acceptable, for instance, a bluish tinge.

The discovery has now been made that the use of a special polymer blend for producing medical devices results in an improved resistance to yellowing when repeated or prolonged exposure to such radiation is made.

The invention relates to the use of a polymer blend for the production of medical devices with improved resistance to yellowing when exposed to sterilization by ionizing radiation, wherein the blend is formed of a first polymer selected from polycarbonate homopolymers, poly(ester carbonates), poly(sulfone carbonates), and wholly aromatic polyesters, and a second polymer selected from polyesters, based on aliphatic, aliphatic ether or cycloaliphatic polyols and aromatic or cycloaliphatic dicarboxylic acids, poly(sulfone carbonates) and mixtures thereof, with the provision that the two polymers are not from the same specific class, wherein the two polymers range in the amount between 99:1 and 1:99 parts by weight, based on 100 parts by weight, of the two combined polymers.

By way of illustration, polymers which are normally susceptible to ionizing radiation-caused yellowing include aromatic (homo)polycarbonates, poly(ester carbonates), poly(sulfone carbonates), and poly(arylates) which are admixed with a second polymer selected from among polyester homopolymers, especially those based on the reaction of aliphatic or cycloaliphatic polyols with aromatic or cycloaliphatic dicarboxylic acids, copolyesters.

The particular pairing in a given case will depend on whether a noticeable improvement in the ionizing radiation resistance and a concomitant reduction in the tendency to undergo yellowing are achieved. For instance, as a bare minimum requirement it is essential that the first and second polymers be of different specific classes, the observation having been made that no appreciable benefit is obtained, if, for instance, a poly(sulfone carbonate) is paired with another poly(sulfone carbonate), a poly(aromatic carbonate) homopolymer paired with another poly(aromatic carbonate) homopolymer, a poly(ester carbonate) is paired with another poly(ester carbonate) and so forth. Below are listed various polymer combinations, or pairings, which are preferred and suggested for use in the practice of the invention:

| FIRST POLYMER | SECOND POLYMER |
|---|---|
| Polycarbonate homopolymer | (a) Polyester homopolymer<br>(b) Copolyester<br>(c) Poly(sulfone-carbonate) |
| Poly(ester-carbonate) | (a) Polyester homopolymer<br>(b) Copolyester<br>(c) Poly(sulfone-carbonate) |
| Polyarylate | (a) Polyester homopolymer<br>(b) Copolyester |

These various polymers and copolymers are described in greater detail below.

Polycarbonate homopolymers useful in this invention are especially aromatic polycarbonates. These can be made by those skilled in the art or obtained from various commercial sources. They may be prepared by reacting dihydric phenol with a carbonate precursor, such as phosgene, a haloformate or a

carbonate ester. Typically, they will have recurring structural units of the formula:

$$-\!\!\left(O-A-O-\overset{\overset{\displaystyle O}{\|}}{C}\right)\!\!-$$

wherein A is a divalent aromatic radical of the dihydric phenol employed in the polymer producing reaction. Preferably, the aromatic carbonate polymers have an intrinsic viscosity ranging from 0.30 to 1.0 dl./g. (measured in methylene chloride at 25°C.) By dihydric phenols is meant mononuclear or polynuclear aromatic compounds containing two hydroxy radicals, each of which is attached to a carbon atom of an aromatic nucleus. Typical dihydric phenols include 2,2-bis-(4-hydroxy-phenyl)propane; 2-2-bis-(3,5-dimethyl-4-hydroxyphenyl)propane, 4,4′-dihydroxydiphenyl ether, bis(2-hydroxyphenyl)methane, mixtures thereof. The preferred aromatic carbonate polymer is a homopolymer derived from 2,2-bis(4-hydroxyphenyl)propane(bisphenol-A).

Poly(ester-carbonates) for use in the invention are known and can be obtained commercially. Generally, they are copolyesters comprising recurring carbonate groups:

$$-\!\!\left(O-\overset{\overset{\displaystyle O}{\|}}{C}-O\right)\!\!- ,$$

carboxylate groups:

$$-\!\!\left(\overset{\overset{\displaystyle O}{\|}}{C}-O\right)\!\!- \text{, and}$$

aromatic carbocyclic groups in the linear polymer chain, in which at least some of the carboxylate groups and at least some of the carbonate groups are bonded directly to ring carbon atoms of the aromatic carbocyclic groups. These poly(ester-carbonate) copolymers, in general, are prepared by reacting a difunctional carboxylic acid, such as phthalic acid, isophthalic acid, terephthalic acid, homophthalic acid, o-, m-, and p-phenylenediacetic acid, the polynuclear aromatic acids, such as diphenic acid, 1,4-naphthalic acid, mixtures of any of the foregoing, and the like, with a dihydric phenol and a carbonate precursor, of the types described above. A particularly useful polyester carbonate is derived from bisphenol-A, isophthalic acid, terephthalic acid, or a mixture of isophthalic acid and terephthalic acid, or the reactive derivatives of these acids such as terephthaloyl dichloride, isophthaloyl dichloride, or a mixture thereof, and phosgene. The molar proportions of dihydroxy diaryl units to benzenedicarboxylate units to carbonate units can range from 1:0.2—1.00:0.80—0.00 and the molar range of terephthalate units to isophthalate units can range from 99:1 to 1:99 in this preferred family of resins. When the molar proportion of carbonate units is 0, the resin is a wholly aromatic polyester. See US—A—4,324,869.

The aromatic dihydric phenol sulfone resins used in this invention are a family of resins which can be made by those skilled in this art. For example, homopolymers of dihydric phenol, and a dihydroxydiphenyl sulfone and a carbonate precursor can be prepared, as well as copolymers of a dihydric phenol and a carbonate precursor can be made according to the description in US—A—3,271,367. A preferred material is made by polymerizing bis-(3,5-dimethyl-4-hydroxy phenyl)sulfone, alone, or especially in combination with bisphenol-A with phosgene or a phosgene precursor, in accordance with the description in US—A—3,737,409. Especially preferred is a copolymer made by reacting 1—99, preferably 40—99 wt. percent of the sulfone, 99 to 1, preferably 60 to 1 wt. percent of the bisphenol with phosgene.

Polyesters suitable for use herein are derived from an aliphatic, aliphatic ether or cycloaliphatic diol, or mixtures thereof, preferably containing from about 2 to about 10 carbon atoms, and one or more aromatic or cycloaliphatic dicarboxylic acids. Preferred polyesters are derived from an aliphatic diol and an aromatic dicarboxylic acid having repeating units of the following general formula:

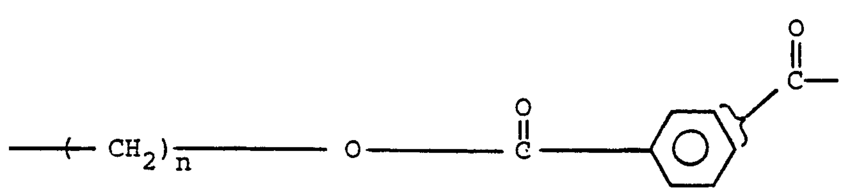

wherein n is an integer of from 2 to 4. The most preferred polyester is poly(ethylene terephthalate).

Also contemplated herein are the above polyesters with additional amounts of polyols and/or acids in the amounts of from 0.5 to 50 wt. percent based on the total composition. The acids can be aliphatic or cycloaliphatic with the number of carbon atoms ranging from 2 to 20. Likewise, the glycols can be

cycloaliphatic or aliphatic with the number of carbon atoms covering the same range. Polyalkylene ether glycols can also be used where the alkylene portion has from 2 to 10 carbon atoms and the entire glycol portion varies in molecular weight from 100 to 10,000. All such polyesters can be made following the teachings of, for example, US—A—2,465,319 and 3,047,539.

The polyesters which are derived from a cycloaliphatic diol and an aromatic dicarboxylic acid are prepared, for example, by condensing either the cis- or trans-isomer (or mixtures thereof) of, for example, 1,4-cyclohexanedimethanol with an aromatic dicarboxylic acid so as to produce a polyester having recurring units of the following formula:

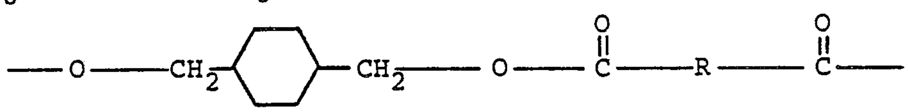

wherein the cyclohexane ring is selected from the cis- and trans-isomers thereof and R represents an aryl or cycloaliphatic radical containing 6 to 20 carbon atoms and which is the decarboxylated residue derived from an aromatic dicarboxylic acid.

Examples of aromatic dicarboxylic acids represented by the decarboxylated residue R are isophthalic or terephthalic acid, 1,2-di(p-carboxyphenyl)ethane, 4,4'-dicarboxydiphenyl ether, etc., and mixtures of these. Acids containing fused rings can also be present, such as in 1,4- or 1,5-naphthalenedicarboxylic acids. Also contemplated are cycloaliphatic diacids, such as cyclohexane dicarboxylic acid. The preferred dicarboxylic acids are terephthalic acid or a mixture of terephthalic and isophthalic acids.

Another preferred polyester may be derived from the reaction of either the cis- or trans-isomer (or a mixture thereof) of 1,4-cyclohexanedimethanol with a mixture of isophthalic and terephthalic acids. Such a polyester would have repeating units of the formula:

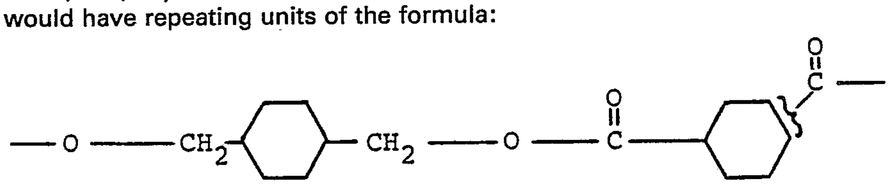

Still another preferred polyester is a copolyester derived from a cyclohexane dimethanol, an alkylene glycol and an aromatic dicarboxylic acid. These copolyesters are prepared by condensing either the cis- or trans-isomer (or mixtures thereof) of, for example, 1,4-cyclohexane-dimethanol and an alkylene glycol with an aromatic dicarboxylic acid so as to produce a copolyester having units of the following formula:

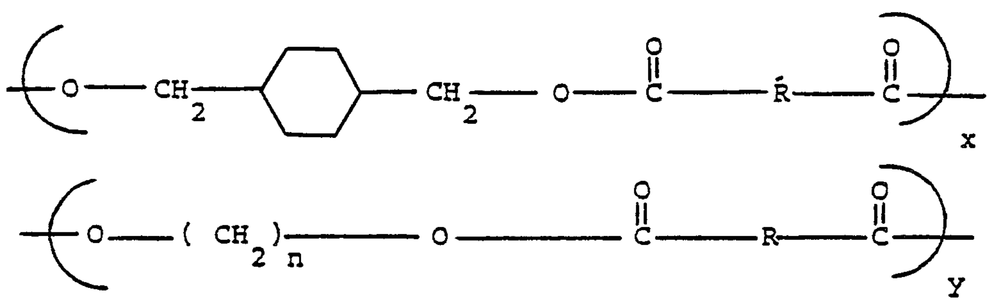

wherein the cyclohexane ring is selected from the cis- and trans-isomers thereof, R is as previously defined, n is an integer of 2 to 10, the x units comprise from about 1 to about 99 percent by weight, and the y units comprise from about 99 to about 1 percent by weight.

Such a preferred copolyester may be derived from the reaction of either the cis- or trans-isomer (or mixtures thereof) of 1,4-cyclohexanedimethanol and ethylene glycol with terephthalic acid in a molar ratio of 80:20:100. These copolyesters have repeating units of the following formula:

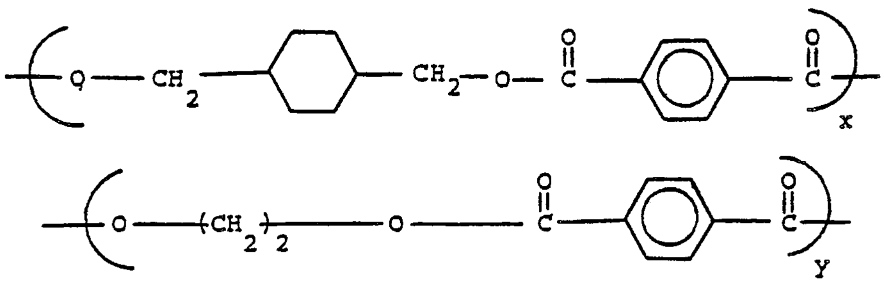

wherein x and y are as previously defined.

The polyesters described herein are either commercially available or they can be produced by methods known in the art, including those set forth in US—A—2,901,466.

The polyesters employed in the practice of this invention will usually have an intrinsic viscosity of from about 0.4 to about 2.0 dl./g., as measured in a 60:40 phenol:tetrachloroethane mixture, or similar solvent at 23°—30°C.

The relative amounts of the polymers can and usually do vary widely in the blend, with particular amounts depending on specific requirements and the nature of the polymers being employed. Proportions range between 99:1 and 1:99, based on 100 parts by weight of the two polymers together. Best amounts in a given instance will be readily determinable by those skilled in the art.

Preparation of admixtures of the polymers may be carried out in any convenient manner. In the usual case, finely divided dry powders of the polymers are simply blended together on a mechanical mixer and the blend is compounded by passage through an extruder at an elevated temperature above the softening points of the polymeric constituents. Alternatively, however, the polymers may be dissolved in a mutual solvent from which they are subsequently recovered in blended form by evaporation, distillation or precipitation.

The resulting polymer blend can be further modified, if desired, by incorporation of standard amounts of conventional additives to upgrade other physical or chemical properties. The additive or additives can be selected from among, by way of illustration, plasticizers, thermal stabilizers, antioxidants, flame retardants, lubricants, fillers, reinforcing agents, and so on. These are most conveniently added during the initial stages of polymer blend formation.

The blends may be subsequently processed into molded articles of various shapes and sizes and they are especially useful in medical products such as those described above. These may be subjected to the rigorous conditions required for sterilization, without any or only small losses in the optical clarity.

The following examples show polymer blends used for producing medical devices.

Examples 1—17

The polymer blends noted below were prepared by forming an admixture, extruding at a temperature of from 260 to 315°C (500 to 600°F) and injection molding into test pieces at a temperature of from 260°C to 304°C (500 to 580°F). The same was done for several of the polymers separately, which are identified as controls. Color change was evaluated using Cobalt-60 as the gamma radiation source, with dosages (in Mrads) as shown. The yellowness index (YI) was measured in accordance with the ASTM procedure. The slopes were calculated from a plot of the delta yellowness index versus the radiation dose. Lower slope values for the blends, in relation to the controls, is indicative of less yellowing. The formulations used and the results obtained are set forth in TABLE 1:

TABLE 1

| Ex. | Base Resin | Additive | Additive Amt., wt.% | After Irradiation | | |
|---|---|---|---|---|---|---|
| | | | | Dose | ΔYI | Slope |
| A* | Poly(bisphenol-A carbonate)[a] | None | 0 | 5.7 | 42.34 | 7.42 |
| 1 | Same | Poly(carbonate-sulfone)[b] | 20 | 5.7 | 21.49 | 3.77 |
| 2 | Same | Poly(carbonate-sulfone)[b] | 50 | 5.7 | 19.06 | 3.34 |
| 3 | Same | Poly(carbonate-sulfone)[b] | 75 | 5.7 | 13.70 | 2.40 |
| B* | None | Poly(carbonate-sulfone)[b] | 100 | 5.2 | 7.1 | 1.36 |
| 4 | Poly(bisphenol A carbonate) | Polyethylene terephthalate | 23 | 5.0 | 22 | 4.4 |
| 5 | Same | Polyethylene terephthalate | 40 | 5.0 | 21 | 4.2 |
| C* | None | Polyethylene terephthalate | 100 | 5.0 | 2 | 0.4 |

TABLE 1 (continued)

| Ex. | Base Resin | Additive | Additive Amt., wt.% | After Irradiation | | |
|---|---|---|---|---|---|---|
| | | | | Dose | ΔYI | Slope |
| 6 | Poly(bisphenol A carbonate) | Copolyester of cyclohexane dimethanol, ethylene glycol and terephthalic acid | 20 | 5.0 | 13 | 2.6 |
| 7 | Same | Same copolyester | 40 | 5.0 | 8 | 1.6 |
| 8 | Same | Same copolyester | 60 | 5.0 | 5.5 | 1.1 |
| 9 | Same | Same copolyester | 80 | 5.0 | 4.0 | 0.8 |
| D* | None | Same copolyester | 100 | 5.0 | 10.0 | 2.0 |
| 10 | Poly(bisphenol-A carbonate) | Polyester of 1,4-cyclohexanedimethanol and 1,4-cyclohexane dicarboxylic acid[c] | 50 | 5.8 | 9.64 | 1.66 |
| 11 | Same | Hydroxy-terminated polyester of ethylene glycol, phthalic acid, and branching agent[d] | 10 | 2.5 | 6.90 | 2.76 |
| 12 | Same | Polyester derived from ethylene, butylene glycol and adipic acid[e] | 10 | 2.5 | 8.68 | 3.47 |

TABLE 1 (continued)

| Ex. | Base Resin | Additive | Additive Amt., wt.% | After Irradiation | | |
|---|---|---|---|---|---|---|
| | | | | Dose | ΔYI | Slope |
| 13 | Same | Copolyester: 85 mole% terephthalate, 15 mole% isophthalate and 1,4-cyclohexane dimethanol[f] | 10 | 5.0 | 14.1 | 2.82 |
| 14 | Same | Copolyester[f] | 20 | 5.0 | 11.1 | 2.22 |
| 15 | Same | Copolyester[f] | 30 | 5.0 | 9.4 | 1.88 |
| 16 | Same | Copolyester[f] | 40 | 5.0 | 7.6 | 1.52 |
| 17 | Same | Copolyester[f] | 50 | 5.0 | 8.0 | 1.60 |
| E* | None | Copolyester[f] | 100 | 5.0 | 7.0 | 1.40 |

* control experiment
[a] LEXAN®, General Electric Co.
[b] Fox, U.S. 3,737,409, Example IV.
[c] PCCE, Eastman Kodak Co.
[d] ADMEX 433, Sherex Chemical Co., Inc.
[e] ADMEX SC 3636—70, Sherex
[f] KODAR, Eastman Kodak Co.

It should be noted that some of these formulations may undergo darkening during high temperature processing and thus their use would probably be restricted to opaque or dark-colored articles.

Example 18

The general procedure of Examples 1—17 was repeated, substituting for the second resin a copolyester comprising units derived from poly(tetramethyleneether)glycol (24 wt.%), 1,4-butanediol (16 wt.%), and neopentyl glycol (4 wt.%), and terephthalic acid (56 wt.%). A small amount of catalyst quenching aid, phosphorus acid, was included. The ratio of polycarbonate to copolyester was 1:1. After exposure to a gamma radiation source and comparison with unmodified polycarbonate exposed to a similar source, there was visually observed hardly any yellowing with the composition of this example. Unmodified polycarbonate yellowed significantly.

Example 19

The general procedure of Examples 1—17 was repeated, and a small amount of a combination of tinting dyes and a small amount of a quenching aid, phosphorous acid, were also included. In addition to exposure to a gamma radiation source, some of the specimens were exposed to a source of electron beam radiation, which yellows unmodified polycarbonate significantly. The formulations employed and the results obtained are set forth in TABLE 2:

TABLE 2

| Example | 19 |
|---|---|
| Composition (parts by weight) | |
| Poly(bisphenol A carbonate)[a] | 50 |
| Copolyester: 85 mole% terephthalate, 15 mole% isophthalate and 1,4-cyclohexane dimethanol[b] | 50 |
| Mixed red and blue dyes | 0.00102 |
| Properties | |
| Gamma irradiated, dosage | Yellowness Index, YI |
| 0 | 0.97 |
| 1.5 megarads | 3.48 |
| 2.5 megarads | 4.85 |
| 5 megarads | 7.48 |
| Electron beam irradiated, dosage | |
| 0 | 1.5 |
| 1.5 megarads | 5.13 |
| 2.5 megarads | 6.96 |
| 3.0 megarads | 6.35 |

[a] LEXAN®, General Electric Co.
[b] KODAR®, Eastman Kodak Co.

The composition of Example 19 was color stabilized against both gamma radiation and electron beam radiation.

Other modifications and variations are possible in the light of the above disclosure. For example, if a polyarylate polymer comprising units derived from bisphenol A and terephthalic acid is substituted for the poly(bisphenol A carbonate) polymer in the composition of Example 19, a composition color stabilized against both gamma radiation and electron beam radiation will be obtained.

**Claims**

1. Use of a blend of a first polymer selected from polycarbonate homopolymers, poly(ester-carbonates), poly(sulfone-carbonates), and wholly aromatic polyesters, and a second polymer selected from polyesters, based on aliphatic, aliphatic ether or cycloaliphatic polyols and aromatic or cycloaliphatic dicarboxylic acids, poly(sulfone-carbonates), and mixtures thereof, with the provision, that the two polymers are not from the same specific class, wherein the two polymers range in the amount between 99:1 and 1:99 parts by weight, based on 100 parts by weight of the two combined polymers, for producing medical devices.

2. Use of a polymer blend according to claim 1, in which the first polymer is a polycarbonate homopolymer.

3. Use of a polymer blend according to claim 2, in which the polycarbonate is aromatic.

4. Use of a polymer blend according to claim 3, in which the aromatic polycarbonate has recurring units of the formula

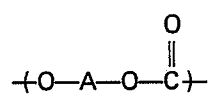

in which A is a divalent aromatic radical of a dihydric phenol.

5. Use of a polymer blend according to claim 1, in which the first polymer is a poly(ester-carbonate).

6. Use of a polymer blend according to claim 5 in which the poly(ester-carbonate) has recurring carbonate groups:

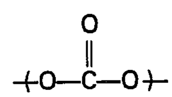 ,

recurring carboxylate groups

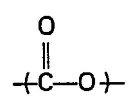 ,

and aromatic carbocyclic groups in the linear polymer chain, in which at least some of the carboxylate groups and at least some of the carbonate groups are bonded directly to ring carbon atoms of the aromatic carbocyclic groups.

7. Use of a polymer blend according to claim 1, in which the second polymer is a polyester homopolymer or copolymer.

8. Use of a polymer blend according to claim 7, in which the polyester is a poly(alkylene aromatic dicarboxylate).

9. Use of a polymer blend according to claim 8, in which the poly(alkylene aromatic dicarboxylate) is derived from an aliphatic diol and an aromatic dicarboxylic acid and has repeating units of the formula

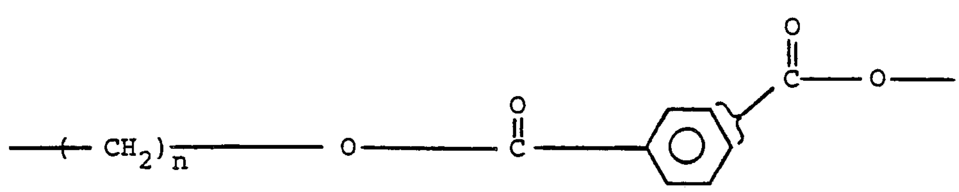

wherein n is an integer from 2 to 4.

10. Use of a polymer blend according to claim 9, in which the poly(alkylene aromatic dicarboxylate) is poly(ethylene terephthalate).

11. Use of a polymer blend according to claim 7, in which the poly(alkylene aromatic dicarboxylate) is derived from a cycloaliphatic diol and an aromatic dicarboxylic acid and has recurring units of the formula

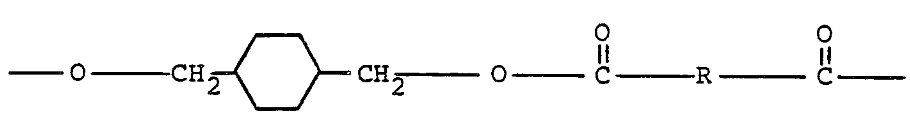

wherein the cyclohexane ring is selected from the cis- and trans-isomers thereof and R represents an aryl or cycloaliphatic radical containing from 6 to 20 carbon atoms and which is the decarboxylated residue derived from an aromatic dicarboxylic acid.

12. Use of a polymer blend according to claim 11, in which the poly(alkylene aromatic dicarboxylate) is derived from the reaction of 1,4-cyclohexanedimethanol with a mixture of isophthalic and terephthalic acids, having repeating units of the formula

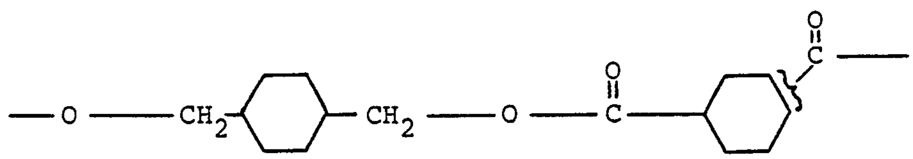

13. Use of a polymer blend according to claim 7, in which, the copolyester is derived from cyclohexane dimethanol, an alkylene glycol and an aromatic dicarboxylic acid, having units of the formula

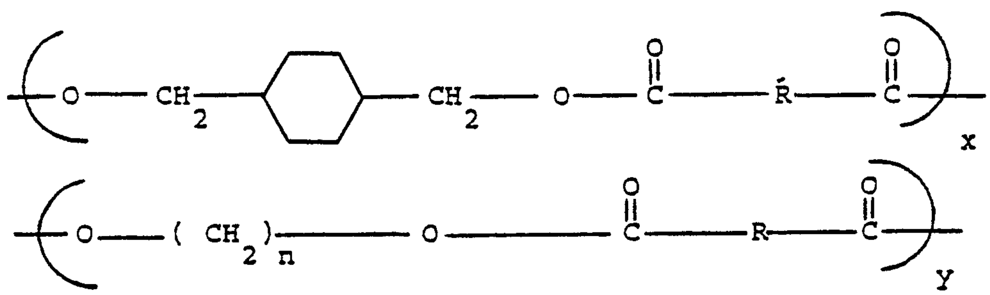

in which the cyclohexane ring is selected from the cis- and trans-isomers thereof, R is the decarboxylated residue of isophthalic or terephthalic acid, n is an integer from 2 to 10, the x units comprise from 1 to 99 percent by weight, and the y units comprise from 99 to 1 percent by weight.

14. Use of a polymer blend according to claim 13, in which the copolyester is derived from the reaction of 1,4-cyclohexane dimethanol and ethylene glycol with terephthalic acid.

15. Use of a polymer blend according to claim 1, in which the first polymer is poly(bisphenol A carbonate) homopolymer and the second polymer is a poly(carbonatesulfone) copolymer.

16. Use of a polymer blend according to claim 1, in which the first polymer is poly(bisphenol A carbonate) homopolymer and the second polymer is poly(ethylene terephthalate).

17. Use of a polymer blend according to claim 1, in which the first polymer is poly(bisphenol A carbonate) homopolymer and the second polymer is a copolyester of 1,4-cyclohexane dimethanol, ethylene glycol and terephthalic acid.

18. Use of a polymer blend according to claim 1, in which the first polymer is poly(bisphenol A carbonate) homopolymer and the second polymer is an hydroxy terminated polyester of ethylene glycol, phthalic acid and a branching agent.

19. Use of a polymer blend according to claim 1, in which the first polymer is poly(bisphenol A carbonate) homopolymer and the second polymer is a polyester of ethylene glycol, butylene glycol and adipic acid.

20. Use of a polymer blend according to claim 1, in which the first polymer is poly(bisphenol A carbonate) homopolymer and the second is a copolyester of terephthalate, isophthalate and 1,4-cyclohexane dimethanol.

21. Use of a polymer blend according to claim 1, in which the first polymer is poly(bisphenol A carbonate) homopolymer and the second polymer is a polyester of 1,4-cyclohexane dimethanol and 1,4-cyclohexane dicarboxylic acid.

22. Use of a polymer blend according to claim 1, in which the first polymer is poly(bisphenol-A carbonate) homopolymer and the second polymer is a copolyester comprising units derived from poly(tetramethylene)glycol, 1,4-butanediol, neopentyl glycol and terephthalic acid.

**Patentansprüche**

1. Verwendung einer Mischung aus einem ersten Polymer ausgewählt aus Polycarbonat Homopolymeren Poly(ester-carbonate) Poly(sulfon-carbonaten) und ganz aromatischen Polyestern und einem zweiten Polymeren ausgewählt aus Polyester basierend auf aliphatischen, aliphatischen Äthern oder cycloaliphatischen Polyolen und aromatischen oder cycloaliphatischen Dicarbonsäuren, Poly(sulfon-carbonaten) und Mischungen derselben mit der Maßgabe, daß die beiden Polymeren nicht von der gleichen speziellen Klasse sind, worin die beiden Polymeren in Mengen vorliegen, die zwischen 99:1 und 1:99 Gewichtsteilen bezogen auf 100 Gewichtsteile der beiden vereinigten Polymeren basieren, zur Erzeugung medizinischer Geräte.

2. Verwendung einer Polymermischung nach Anspruch 1, in welcher das erste Polymere ein Polycarbonat Homopolymer ist.

3. Verwendung einer Polymermischung nach Anspruch 2, in welcher das Polycarbonat aromatisch ist.

4. Verwendung einer Polymermischung nach Anspruch 3, in welcher das aromatische Polycarbonat wiederkehrende Einheiten der Formel

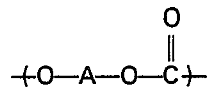

aufweist, worin A ein zweiwertiger aromatischer Rest eines zweiwertigen Phenols ist.

5. Verwendung einer Polymermischung nach Anspruch 1, in welcher das erste Polymere ein Poly(ester-carbonat) ist.

6. Verwendung einer Polymermischung nach Anspruch 5, in welcher das Poly(ester-carbonat) wiederkehrende Carbonatgruppen:

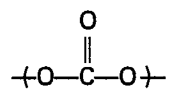

,

wiederkehrende Carboxylatgruppen:

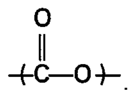

und aromatische carbozyklische Gruppen in der linearen Polymerkette aufweist, in welcher wenigstens einige der Carboxylatgruppen und wenigstens einige der Carbonatgruppen direkt an Ringkohlenstoffatome und aromatischen carbocyclischen Gruppen gebunden sind.

7. Verwendung einer Polymermischung nach Anspruch 1, in welcher das zweite Polymere ein Polyester Homopolymer oder Copolymer ist.

8. Verwendung einer Polymermischung nach Anspruch 7, in welcher der Polyester ein Poly(alkylene-aromatisches-dicarboxylat) ist.

9. Verwendung einer Polymermischung nach Anspruch 8, in welcher das Poly(alkylen-aromatische-dicarboxylat) abgeleitet ist von einem aliphatischen Diol und einer aromatischen Dicarbonsäure und wiederkehrende Einheiten der Formel

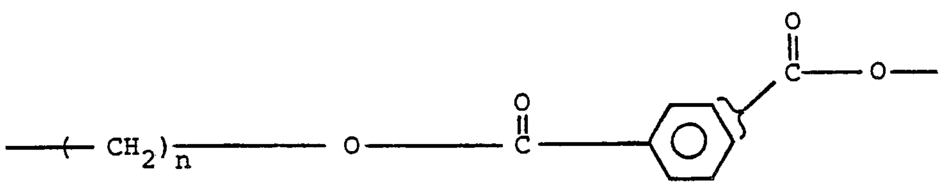

aufweist, worin n eine ganze Zahl von 2 bis 4 ist.

10. Verwendung einer Polymermischung nach Anspruch 9, in welcher das Poly(alkylen-aromatische-dicarboxylat) ein Poly(äthylen-terephthalat) ist.

11. Verwendung einer Polymermischung nach Anspruch 7, in welcher das Poly(alkylen-aromatische-dicarboxylat) abgeleitet ist von einem cycloaliphatischen Diol und einer aromatischen Dicarbonsäure und wiederkehrende Einheiten der Formel

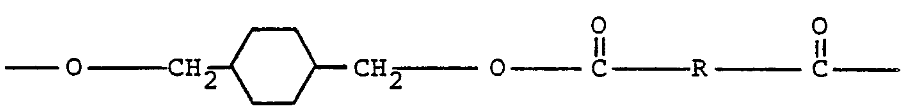

aufweist, worin der Zyklohexanring ausgewählt ist aus den Cis- und Transisomeren derselben und R einen Aryl- oder cycloaliphatischen Rest mit 6 bis 20 Kohlenstoffatomen darstellt und worin der decarboxylierte Rest von einer aromatischen Dicarbonsäure abgeleitet ist.

12. Verwendung einer Polymermischung nach Anspruch 11, in welcher das Poly(alkylen-aromatische-dicarboxylat) abgeleitet ist, von der Reaktion das 1,4-Cyclohexandimethanols mit einer Mischung aus Isophthalsäure und Terephthalsäure und die folgenden wiederkehrenden Einheiten der Formel

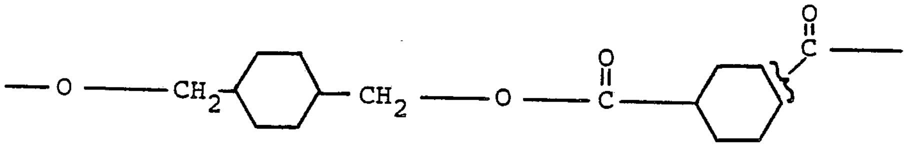

aufweist.

13. Verwendung einer Polymermischung nach Anspruch 7, in welcher der Copolyester abgeleitet ist von Cyclohexandimethanol, einem Alkylenglykol und einer aromatischen Dicarbonsäure welche Einheiten der Formel

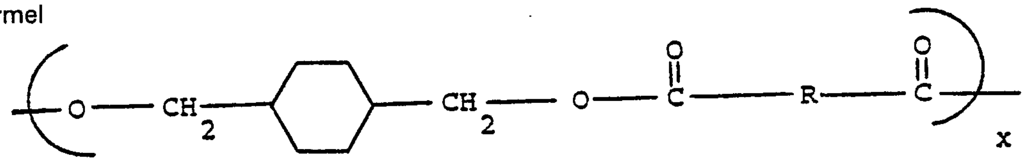

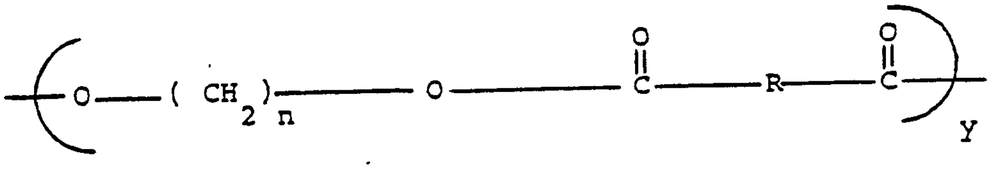

aufweist, in welcher der Cyclohexanring ausgewählt ist aus den Cis- und Transisomeren derselben, R der decarboxylierte Rest der Isophtalsäure oder Terephtalsäure ist, n eine ganze Zahl von 2 bis 10 ist, die x Einheiten von 1 bis 99 Gew.-% und die y Einheiten von 99 bis 1 Gew.-% ausmachen.

14. Verwendung einer Polymermischung nach Anspruch 13, in welcher der Copolyester abgeleitet ist von der Reaktion des 1,4-Cyclohexandimethanols und des Äthylenglykols mit Terephtalsäure.

15. Verwendung einer Polymermischung nach Anspruch 1, in welcher das erste Polymere Poly(bisphenol-A-carbonat)-Homopolymer und das zweite Polymere ein Poly(carbonatsulfon)-copolymer ist.

16. Verwendung einer Polymermischung nach Anspruch 1, in der das erste Polymere Poly(bisphenol-A-carbonat)-Homopolymer und das zweite Polymere Poly(äthylen-terephtalat) ist.

17. Verwendung einer Polymermischung nach Anspruch 1, in welcher das erste Polymere Poly(bisphenol-A-carbonat)-Homopolymer und das zweite Polymere ein Copolyester des 1,4-Cyclohexan-dimethanols, Ethylen-glykols und Terephtalsäure ist.

18. Verwendung einer Polymermischung nach Anspruch 1, in welcher das erste Polymere Poly(bisphenol-A-carbonat)-Homopolymer und das zweite Polymere ein mit Hydroxy endender Polyester aus Äthylenglykol, Phthalsäure und einem Verzweigungsmittel ist.

19. Verwendung einer Polymermischung nach Anspruch 1, in welcher das erste Polymere Poly(bisphenol-A-carbonat)-Homopolymer und das zweite Polymere ein Polyester aus Äthylenglykol, Butylenglykol und Adipinsäure ist.

20. Verwendung einer Polymermischung nach Anspruch 1, in welcher das erste Polymere Poly(bisphenol-A-carbonat)-Homopolymer und das zweite ein Copolyester des Terephthalats, Isophthalats und 1,4-Cyclohexan-dimethanols ist.

21. Verwendung einer Polymermischung nach Anspruch 1, in welcher das erste Polymere Poly(bisphenol-A-carbonat)-Homopolymer und das zweite Polymere ein Polyester aus 1,4-Cyclohexandimethanol und 1,4-Cyclohexan-dicarbonsäure ist.

22. Verwendung einer Polymermischung nach Anspruch 1, in welcher das erste Polymere Poly(bisphenol-A-carbonat)-Homopolymer und das zweite Polymere ein Copolyester ist, der Einheiten enthält, die von Poly(tetramethylen) glykol 1,4-Butandiol, Neopentyl-glycol und Terephthalsäure abgeleitet sind.

## Revendications

1. Utilisation d'un mélange d'un premier polymère choisi parmi des homopolymères de polycarbonate, des poly(ester-carbonate)s, des poly(sulfone-carbonate)s et des purs polyesters aromatiques et d'un second polymère choisi parmi des polyesters, à base de polyols aliphatiques, d'étherpolyols aliphatiques ou de polyols cycloaliphatiques et d'acides dicarboxyliques aromatiques ou cycloaliphatiques, des poly(sulfone-carbonate)s et leurs mélanges, à condition que les deux polymères ne soient pas du même groupe particulier, dans lequel les deux polymères sont présents en une quantité comprise entre 99:1 et 1:99 parties en poids pour 100 parties en poids des deux polymères combinés, pour la production d'appareils médicaux.

2. Utilisation d'un mélange de polymères selon la revendication 1, dans lequel le premier polymère est un homopolymère de polycarbonate.

3. Utilisation d'un mélange de polymères selon la revendication 2, dans lequel le polycarbonate est aromatique.

4. Utilisation d'un mélange de polymères selon la revendication 3, dans lequel le polycarbonate aromatique a des mailles répondant à la formule:

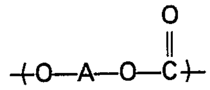

dans laquelle A représente un radical aromatique divalent d'un diphénol.

5. Utilisation d'un mélange de polymères selon la revendication 1, dans lequel le premier polymère est un poly(ester-carbonate).

6. Utilisation d'un mélange de polymères selon la revendication 1, dans lequel le poly(ester-carbonate) a des groupes carbonates récurrents:

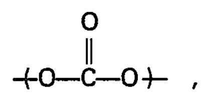

des groupes carboxylates récurrents

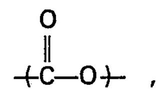

et des groupes carbocycliques aromatiques dans la chaîne linéaire du polymère, sachant qu'au moins certains des groupes carboxylates et au moins certains des groupes carbonates sont liés directement aux atomes de carbone des cycles des groupes carbocycliques aromatiques.

7. Utilisation d'un mélange de polymères selon la revendication 1, dans lequel le second polymère est un homopolymère ou un copolymère de polyester.

8. Utilisation d'un mélange de polymères selon la revendication 7, dans lequel le polyester est un poly(dicarboxylate aromatique d'alkylène).

9. Utilisation d'un mélange de polymères selon la revendication 8, dans lequel le poly(dicarboxylate aromatique d'alkylène) est dérivé d'un diol aliphatique et d'un acide dicarboxylique aromatique et a des motifs répétés répondant à la formule

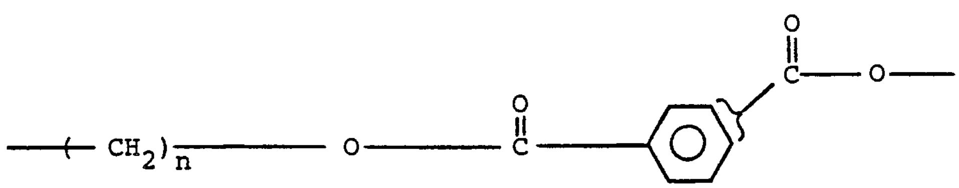

dans laquelle n représente un nombre entier compris entre 2 et 4.

10. Utilisation d'un mélange de polymères selon la revendication 9, dans lequel le poly(dicarboxylate aromatique d'alkylène) est le poly(téréphtalate d'éthylène).

11. Utilisation d'un mélange de polymères selon la revendication 7, dans lequel le poly(dicarboxylate aromatique d'alkylène) est dérivé d'un diol cycloaliphatique et d'un acide dicarboxylique aromatique et a des motifs récurrents répondant à la formule

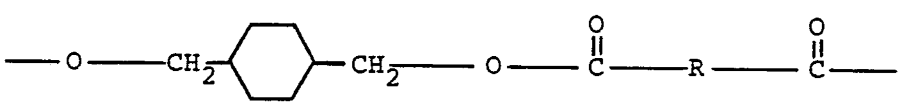

dans laquelle le cycle cyclohexane est l'isomère cis ou l'isomère trans et R représente un radical aryle ou cycloaliphatique contenant de 6 à 20 atomes de carbone et qui est le reste décarboxylé dérivé d'un acide dicarboxylique aromatique.

12. Utilisation d'un mélange de polymères selon la revendication 11, dans lequel le poly(dicarboxylate aromatique d'alkylène) est dérivé de la réaction du di(hydroxyméthyl)-1,4 cyclohexane et d'un mélange d'acides isophtalique et téréphtalique, ayant des motifs répétés répondant à la formule

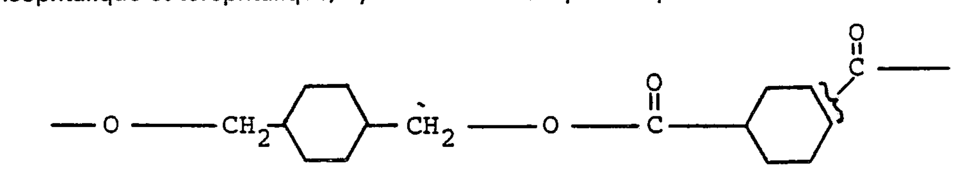

13. Utilisation d'un mélange de polymères selon la revendication 7, dans lequel le copolyester est dérivé de di(hydroxyméthyl) cyclohexane, d'un alkylène-glycol et d'un acide dicarboxylique aromatique, ayant des motifs répondant à la formule

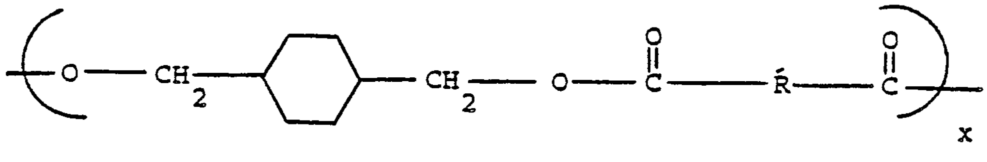

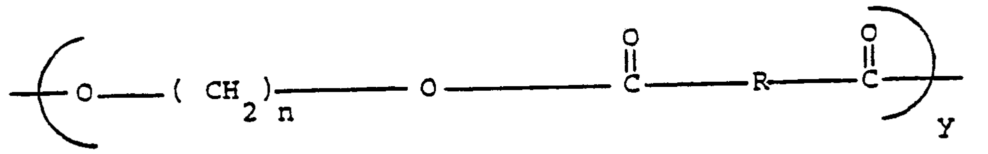

dans laquelle le cycle cyclohexane est l'isomère cis ou l'isomère trans, R représente le reste décarboxylé de l'acide isophtalique ou téréphtalique, n représente un nombre entier compris entre 2 et 10, les x motifs représentent de 1 à 99% en poids et les y motifs représentent de 99 à 1% en poids.

14. Utilisation d'un mélange de polymères selon la revendication 13, dans lequel le copolyester est dérivé de la réaction de di(hydroxyméthyl-1,4 cyclohexane et d'éthylène-glycol avec de l'acide téréphtalique.

15. Utilisation d'un mélange de polymères selon la revendication 1, dans lequel le premier polymère est un homopolymère de poly(carbonate di bisphénol-A) et le second polymère est un copolymère de poly(carbonate-sulfone).

16. Utilisation d'un mélange de polymères selon la revendication 1, dans lequel le premier polymère est un homopolymère de poly(carbonate de bisphénol-A) et le second polymère est un poly(téréphtalate d'éthylène).

17. Utilisation d'un mélange de polymères selon la revendication 1, dans lequel le premier polymère est un homopolymère de poly(carbonate de bisphénol-A) et le second polymère est un copolyester de di(hydroxyméthyl)-1,4 cyclohexane, d'éthylène-glycol et d'acide téréphtalique.

18. Utilisation d'un mélange de polymères selon la revendication 1, dans lequel le premier polymère est un homopolymère de poly(carbonate de bisphénol-A) et le second polymère est un polyester d'éthylène-glycol, d'acide phtalique et d'un agent de ramification, à terminaisons hydroxy.

19. Utilisation d'un mélange de polymères selon la revendication 1, dans lequel le premier polymère est un homopolymère de poly(carbonate de bisphénol-A) et le second polymère est un polyester d'éthylène-glycol, de butylène-glycol et d'acide adipique.

20. Utilisation d'un mélange de polymères selon la revendication 1, dans lequel le premier polymère est un homopolymère de poly(carbonate de bisphénol-A) et le second est un copolyester de téréphtalate, d'isophtalate et de di(hydroxyméthyl)-1,4 cyclohexane.

21. Utilisation d'un mélange de polymères selon la revendication 1, dans lequel le premier polymère est un homopolymère de poly(carbonate de bisphénol-A) et le second polymère est un polyester de di(hydroxyméthyl)-1,4 cyclohexane et d'acide cyclohexane-dicarboxylique-1,4.

22. Utilisation d'un mélange de polymères selon la revendication 1, dans lequel le premier polymère est un homopolymère de poly(carbonate de bisphénol-A) et le second polymère est un copolyester comprenant des motifs dérivés de poly(tétraméthylène-glycol), de butane-diol-1,4, de néopentyl-glycol et d'acide téréphtalique.